# EUROPEAN PATENT APPLICATION

(11) **EP 4 151 148 A1**
(43) Date of publication of application: **22.03.2023**
(21) Application number: 21197565.1
(22) Date of filing: 17.09.2021
(51) Int. Cl.: A61B 5/0295, A61B 5/0507, A61B 5/08, A61B 5/113, G16H 50/20, A61B 5/00, A61B 5/026, A61B 5/029, A61B 5/055, A61B 5/0205, A61B 5/11, A61B 5/02

(54) **SYSTEM FOR DETERMINING A PHYSIOLOGICAL PARAMETER OF A SUBJECT**

(71) Applicant: UMC Utrecht Holding B.V., 3584 CS Utrecht (NL)
(72) Inventor: van den Berg, Cornelis A.T., 3584 CX Utrecht (NL); Steensma, Bart R., 3584 CX Utrecht (NL)
(74) Representative: Eisenführ Speiser

(57) **Abstract**

The invention relates to a system 1 for determining a physiological parameter like a stroke volume of the heart of a subject 7. A measurement device is worn and includes a) an RF antenna module 3 with one or more RF antennas 4, 5 and b) an RF instrument 2 like a vector network analyzer configured to transmit RF power into the RF antenna module, to receive an RF signal from the RF antenna module and to provide a motion signal that is related to a mechanical movement of a structure within the subject based on the received RF signal. The physiological parameter is determined based on the provided motion signal and a model that provides, as an output, the physiological parameter if, as an input, the motion signal is provided.

## Description

### FIELD OF THE INVENTION

The invention relates to a system, method and computer program for determining a physiological parameter of a subject. The invention relates further to a measurement device configured to be worn by a subject and a determination device for determining a physiological parameter of a subject, wherein the measurement device is configured to provide a motion signal and the determination device is configured to determine the physiological parameter based on the provided motion signal. The invention also relates to a training system, training method and training computer program for training a model to be used by the determination device for determining the physiological parameter of the subject. The physiological parameter preferentially is a heart-related physiological parameter or a lung-related physiological parameter.

### BACKGROUND OF THE INVENTION

The articles "Wearable radio-frequency sensing of respiratory rate, respiratory volume, and heart rate" by P. Sharma et al., npj Digital Medicine 3, volume 98, pages 1 to 10 (2020) and "Microwave apexcardiography" by J. Lin et al., IEEE T-MTT 6, volume 27, pages 618 to 620 (1979) disclose systems for determining respiratory rate, respiratory volume and heart rate, wherein the systems use wearable radiofrequency (RF) sensing devices for determining these physiological parameters. The used sensors can be over-clothing wearable RF sensors.

### SUMMARY OF THE INVENTION

It is an object of the present invention to provide a system, method and computer program which allow for an improved determination of a physiological parameter of a subject. It is a further object of the present invention to provide a measurement device configured to be worn by the subject and a determination device for determining a physiological parameter of the subject, wherein the determination device is configured to allow for an improved determination of the physiological parameter based on a motion signal provided by the measurement device. Moreover, the invention relates to a training system, method and computer program for training a model to be used by the system and the determination device for allowing for the improved determination of the physiological parameter.

In a first aspect of the present invention a system for determining a physiological parameter of a subject is presented, wherein the system comprises:
- a measurement device configured to be worn by the subject and including a) an RF antenna module comprising one or more RF antennas and b) an RF instrument connected to the RF antenna module and configured to transmit RF power into the RF antenna module, to receive an RF signal from the RF antenna module and to provide a motion signal that is related to a mechanical movement of a structure within the subject based on the received RF signal,
- a determination device configured to determine the physiological parameter based on the provided motion signal, wherein the determination device comprises a model providing module configured to provide a model that provides, as an output, a physiological parameter if, as an input, a motion signal is provided, and a processor configured to determine the physiological parameter based on the provided model and the provided motion signal.

Since the RF instrument and the RF antenna module are configured to provide a motion signal that is related to a mechanical movement of a structure like an organ within the subject, i.e. since the RF instrument and the RF antenna module are configured such that the measurement region, which is the region in which the measurement device can sense mechanical motion, covers the structure, the mechanical movement of the structure within the subject directly influences the provided motion signal. Moreover, since this directly influenced motion signal is used by the determination device for determining the physiological parameter, the physiological parameter can be determined with an increased sensitivity.

This is in contrast to the measurement described in the above mentioned articles by P. Sharma et al. and J. Lin et al., in which the measured signal is related to electrical changes close to the skin surface only, i.e. it is not related to mechanical motion of a structure within the subject. For instance, if the structure is the heart, the RF radiation does not penetrate into the heart due to the high RF transmit frequency, which limits the sensitivity. Furthermore, the measurements described in these articles do not allow to determine many different cardiac parameters. For instance, it is not possible to quantify stroke volume of the heart with the measurements disclosed in these articles.

The RF instrument can be configured to directly provide the received RF signal as the motion signal. However, the RF instrument can also be configured to process the received RF signal and to provide the processed RF signal as the motion signal. Moreover, the RF instrument preferentially is configured to separate the received RF signal from the transmitted RF signal, i.e. from the RF power transmitted into the RF antenna module, if required. In a preferred embodiment the RF instrument is a vector network analyzer. Moreover, in an embodiment the RF instrument is configured to use a bi-directional coupler or to carry out the transmitting and receiving procedure after each other, in order to separate the transmitted RF signal from the received RF signal.

Preferentially, the processor is configured to determine at least one of a heart-related physiological parameter and a lung-related physiological parameter. For instance, the processor can be configured to determine at least one of the heart rate and the stroke volume as the heart-related physiological parameter. Moreover, the processor can be configured to determine at least one of the respiratory rate and the tidal volume as the lung-related physiological parameter.

The measurement device preferentially comprises a transmitter configured to transmit the measured signal to the determination device. In particular, the measurement device and the determination device are separate devices which are connected via a wireless data connection like Bluetooth.

In a preferred embodiment, the model providing module is a storage like a storage of a mobile device or of a personal computer, in which the model is stored and from which the model can be obtained, and the processor can be a processor of the mobile device or of the personal computer, respectively. The mobile device can be, for instance, a smartphone, a tablet computer or a laptop.

The model providing module can be a storage, as mentioned above, in which the model is stored and from which the model can be obtained, but the model providing module can also be a receiving unit configured to receive the model from another device like another storage. It is also possible that the model providing module is configured to generate the model or adapt a present model by training or calibration and to provide the created or adapted model to the processor.

In an embodiment the RF instrument, which in a preferred embodiment is a vector network analyzer, is configured to provide as the motion signal a complex signal. In particular, the RF instrument is configured to provide at least one of a) a complex reflection coefficient and b) a complex coupling coefficient as the motion signal. In a preferred embodiment, the processor is configured to identify a first subsignal of the complex signal having a distinct phase shift of, for example, 90 degrees with respect to a second subsignal of the complex signal and to determine the physiological parameter based on at least one of the identified subsignals, for example based on the first subsignal. It has been found that the complex signal can comprise contributions from at least two subsignals having a distinct phase shift relative to each other of, for instance, 90 degrees. In particular, one of these subsignals can be caused by cardiac motion and the other of these subsignals can be caused by respiratory motion. Thus, by identifying the first subsignal and using the identified first subsignal for determining a heart-related physiological parameter, the determination of the heart-related physiological parameter can be less influenced by respiratory motion, thereby allowing for an increased accuracy of determining the heart-related physiological parameter. Preferentially, the distinct phase is a predetermined phase, wherein the predetermined phase can be predetermined by, for example, a calibration procedure.

In a preferred embodiment the processor is configured to apply a blind source separation technique to the first subsignal and to use the resulting first subsignal to determine the physiological parameter. For instance, independent component analysis or second order blind analysis can be used as blind source separation technique. Moreover, the processor can be configured to apply a frequency filtering to the first subsignal and to use the resulting first subsignal to determine the physiological parameter. The frequency filtering can be, for instance, a band-pass filtering, a low-pass filtering, a high-pass filtering or a Kalman filtering. This further processing of the measured signal finally allows for a further increased accuracy of determining the physiological parameter.

It is also possible that the processor applies at least one of the blind source separation and the frequency filtering to the signal without a previous identification of a first subsignal of the complex signal having a distinct phase shift of, for instance, 90 degrees with respect to a second subsignal of the complex signal, i.e. without a previous distinct phase separation like a 90 degrees separation. Generally, one, two or three of the distinct phase separation, blind source separation and frequency filtering can be used for processing the signal received from the measurement device.

Preferentially, the model providing module is configured to provide a linear model as the model. It has been found that already a linear model can lead to a determination of a physiological parameter like a stroke volume or a heart rate with an increased accuracy such that relatively low computational efforts are required for, for instance, training the model and utilizing the model.

Furthermore, preferentially the RF instrument, particularly the vector network analyzer, and the RF antenna module are configured to be operated in a frequency range from 30 to 1000 MHz, further preferred in a frequency range from 300 to 800 MHz. Moreover, in a preferred embodiment the RF instrument and the RF antenna module are configured to be operated in a frequency range from 30 to 300 MHz. If an operating frequency within this frequency range is used, the RF radiation causes power deposition throughout the whole body including the structure like the heart. Thus, this frequency range can be used to generate a signal related to the mechanical motion of the structure.

In an embodiment the RF instrument and the RF antenna are configured to be operated with an operating frequency of 64, 128 or 300 MHz. These operating frequencies are equal to the Larmor frequency of a magnetic resonance imaging (MRI) system at 1.5 T, 3 T and 7 T, respectively. Thus, as it will be explained further below, if one of these operating frequencies is used, the model can be trained very effectively by using an MRI system having a main magnetic field strength of 1.5 T, 3.0 T or 7.0 T, respectively.

As mentioned above, in an embodiment the RF instrument and the RF antenna are configured to be operated in a frequency range from 300 to 800 MHz. An operating frequency within this frequency range can be advantageous, if a single smaller structure like a smaller organ such as the heart should be monitored, i.e., for instance, if a motion signal related to a mechanical movement of the heart should be provided, because in this case the RF radiation does not cause power deposition throughout the whole body, but only in a region closer to the RF antenna module like a heart region if the RF antenna module is arranged on the subject's breast region.

In an embodiment the RF instrument is configured to transmit RF power into the RF antenna module with different frequencies, in order to provide the motion signal for the different frequencies, wherein the determination device is configured to determine the physiological parameter based on the motion signal provided for the different frequencies. Thus, measurements can be performed, wherein the operating frequency is changing in time. In particular, a frequency sweep can be performed. By measuring at multiple frequencies, both global and local motion effects can be distinguished, which can improve the accuracy of determining the physiological parameter. In an embodiment, different motion signals measured at different frequencies can be used to measure different physiological parameters, for example heart rate, stroke volume and tidal volume are measured at different frequencies. In another embodiment, measurements at multiple frequencies can be combined, for example by averaging, to improve the accuracy of measuring a physiological parameter of interest.

Preferentially, the RF antenna module has a width and a length which are each smaller than 20 cm. In particular, the dimensions of the RF antenna module are such that it can be arranged within a virtual sphere having a diameter of 20 cm. Thus, preferentially the RF antenna module is not too large, in order to allow the RF antenna module to be relatively easily integrated into the measurement device to be worn, particularly into a holder for holding the RF antenna module.

Moreover, preferentially the RF antenna module comprises one or more dipole antennas or loop coils as the one or more RF antennas. Dipole antennas have the benefit that they have a localized sensitivity and their electric field radiates deeply into tissue, which can allow for a further improved accuracy of determining the physiological parameter. Loop coils have the benefits that they can be made very small, even for low transmit frequencies, which allows for an improved integration of the RF antenna module into the measurement device, particularly into a holder of the measurement device.

In an embodiment, a) the RF antenna module comprises one RF antenna and the measurement device is configured such that, if the measurement device is worn by an adult, a center point of the RF antenna is positioned left to the sternum, or b) the RF antenna module comprises at least two RF antennas, in particularly only two RF antennas, and the measurement device is configured such that, if the measurement device is worn by an adult, a center point of one of the at least two RF antennas is positioned left to the sternum and a center point of the other of the at least two RF antennas is positioned right to the sternum. In a preferred embodiment in which the RF antenna module comprises a single RF antenna only, it is positioned with a shift within the range of 2 cm to 4 cm, further preferred with a shift of 3 cm, to the left of the sternum. This allows to position the RF antenna directly above the heart, thereby allowing to determine a heart-related physiological parameter very accurately like the heart rate or the stroke volume. In a further preferred embodiment in which the RF antenna module comprises at least two RF antennas, particularly only two RF antennas, one of the RF antennas is positioned with a shift within the range of 2 cm to 4 cm, further preferred with a shift of 3 cm, to the left of the sternum and the other of the RF antennas is positioned with a shift within the range of 2 cm to 4 cm, further preferred with a shift of 3 cm, to the right of the sternum. If such a configuration is used, one of the RF antennas is located directly above the heart, wherein the signal generated by using this RF antenna can be used for very accurately determining a heart-related physiological parameter. The other RF antenna has a relatively large distance to the heart and is located directly above the lungs such that the signal generated by using the other RF antenna can be indicative of the influence of the movement of the lungs on the signal which has been generated by the RF antenna located directly above the heart. The signal generated by the other RF antenna can hence be used for correcting the signal generated by using the RF antenna directly above the heart such that the influence of the motion of the lungs can be removed or at least reduced in the signal measured by using the RF antenna above the heart. The signal correction can be performed by using digital signal processing techniques to separate the heart and lung motion from the set of received signals, for example principle component analysis, independent component analysis, or other blind source separation techniques. This can lead to a further increased accuracy of determining a heart-related physiological parameter.

Moreover, in a preferred embodiment in which the RF antenna module comprises two RF antennas only, the RF instrument and the two RF antennas are configured such that an inter-element coupling between the two RF antennas is below a predefined value, wherein this predefined value might be, for instance, -12 dB. In particular, the distance between the two RF antennas is such that the inter-element coupling between the two RF antennas is below -12 dB. This distance can be, for instance, within a range of 4 to 8 cm and preferentially is 6 cm, wherein the distance refers to the distance between the center positions of the two RF antennas. This can lead to a further increased accuracy of determining the physiological parameter.

In an embodiment the RF antenna module includes several RF antennas having different transmit phases defining a sensitivity profile of the RF antenna module, wherein the RF antenna module is configured such that the sensitivity profile has its largest sensitivity at the location of the structure which preferentially is an organ. Also this allows for a further increased accuracy of determining the physiological parameter.

In an embodiment the RF antenna module comprises several RF antennas which are arranged in a belt-like configuration. In particular, the belt-like configuration can be such that the RF antennas are arranged around a torso of the subject. Preferentially, the number of RF antennas in the belt-like configuration is within a range from 3 to 32 RF antennas. This configuration can allow to improve the sensitivity of the measurement device to the physiological parameter. In particular, if several RF antennas are arranged in a belt-like configuration around the structure, a sensitivity profile of the RF antenna module comprising the several RF antennas can be adapted such that a very focused maximum sensitivity is at the location of the structure, thereby allowing for a further increased accuracy of determining the physiological parameter. For example, the phase of the signals transmitted to the RF antennas can be modified in such a way that the sensitivity to the physiological parameter is as high as possible. Optimal transmit phases can be determined by comparing measurements that use different transmit phases or by performing an electromagnetic simulation of the full measurement system. The electromagnetic simulations can be performed with different transmit phases to determine optimal measurement settings. The RF antennas of the belt-like configuration receive a multitude of signals. To determine the physiological parameter from these multiple signals, digital signal processing techniques such as principle component analysis, independent component analysis or other blind source separation techniques can be used to separate different contributions to the signals and isolate measurements of physiological parameters.

Preferentially, the measurement device includes a wearable holder comprising at least the one or more RF antennas of the RF antenna module. Moreover, preferentially, the one or more RF antennas of the RF antenna module are flexible. Due to the flexibility of the one or more RF antennas, they can be better integrated into the wearable holder and wearing the measurement device is more convenient for the subject. The wearable holder can comprise a visible marker assigned to an anatomical feature of the subject, wherein the wearable holder can be configured to be worn such that the marker is arranged at a position on the subject at which the assigned anatomical feature is located. In a preferred embodiment, the anatomical feature is the sternum at the height of the nipples. This ensures that the measurement device is worn correctly by the subject such that the one or more RF antennas are positioned at locations on the subject, which allow for the accurate determination of the physiological parameter.

In an embodiment the RF antenna module includes at least a first RF antenna and a second RF antenna, wherein the RF instrument and the RF antenna module are configured to provide a first motion signal of the first RF antenna that is related to a mechanical movement of a first structure within the subject and to provide a second motion signal of the second RF antenna that is related to a mechanical movement of a second structure within the subject, wherein the processor is configured to remove contributions of the movement of the second structure to the first motion signal from the first motion signal based on the provided first and second motion signals and to determine the physiological parameter based on the first motion signal. In particular, the processor is configured to determine a coupling between the first RF antenna and the second RF antenna. The coupling between the first RF antenna and second RF antenna can be determined by measuring the signal scattered from the first antenna to the second antenna. This can be performed with an RF instrument, preferentially with a vector network analyzer, that has two connecting ports, where one port is able to transmit signal into the first RF antenna and the other port receives signal from the second RF antenna. The coupling signal can be used to remove contributions of the movement of the second structure to the first motion signal from the first motion signal based on the determined coupling and to determine the physiological parameter based on the first motion signal. To separate the first motion and the second motion, digital signal processing techniques such as principal component analysis, independent component analysis or other blind source separation techniques can be used. Also this allows for a further increased accuracy of determining the physiological parameter.

In a further aspect of the present invention a measurement device configured to be worn by a subject is presented, wherein the measurement device is configured to be used with the determination device for forming the system for determining a physiological parameter, wherein the measurement device includes a) the RF antenna module comprising the one or more RF antennas and b) the RF instrument connected to the RF antenna module and configured to transmit RF power into the RF antenna module, to receive an RF signal from the RF antenna module and to provide a motion signal that is related to the mechanical movement of the structure within the subject.

In another aspect of the present invention a determination device for determining a physiological parameter of a subject based on a motion signal provided by the measurement device is presented, wherein the determination device comprises the model providing module configured to provide the model that provides, as an output, a physiological parameter if, as an input, a motion signal is provided, and the processor configured to determine the physiological parameter based on the provided model and the provided motion signal.

In a further aspect of the present invention a training system for training a model to be used by the system for determining the physiological parameter of the subject is presented, wherein the training system comprises:
- a training physiological parameter measurement device for measuring a training physiological parameter of a subject,
- a model providing module configured to provide an adaptable model to be trained, wherein the model provides, as an output, a physiological parameter if, as an input, a motion signal is provided,
- an RF antenna module comprising one or more RF antennas and an RF instrument connected to the RF antenna module and configured to transmit RF power into the RF antenna module, to receive an RF signal from the RF antenna module and to provide a motion signal that is related to a mechanical movement of a structure within the subject based on the received RF signal, if the RF antenna module is arranged on the subject, and
- a training module configured to a) determine a physiological parameter of the subject based on the model to be trained and a motion signal provided by the RF instrument and the RF antenna module and b) modify the model such that a deviation between the determined physiological parameter and the training physiological parameter is reduced.

Preferentially, the training physiological parameter measurement device is configured to use the RF antenna module for measuring the training physiological parameter of the subject. This allows to train the model and finally determine the physiological parameter with an even further increased accuracy, because the same RF antenna module can be used for determining the physiological parameter of the subject based on the model and the provided motion signal and for determining the training physiological parameter.

In another aspect of the present invention a method for determining a physiological parameter of a subject is presented, wherein the method comprises:
- providing a motion signal that is related to a mechanical movement of a structure within the subject by using an RF instrument and an RF antenna module of a measurement device as defined by claim 9,
- providing a model that provides, as an output, a physiological parameter if, as an input, a motion signal is provided, by a model providing module, and
- determining the physiological parameter based on the provided model and the provided motion signal by a processor.

In a further aspect of the present invention a training method for training a model to be used by the system for determining a physiological parameter of a subject, particularly as defined by any of claims 1 to 8, is presented, wherein the training method comprises:
- providing a model to be trained by a model providing module, wherein the model provides, as an output, a physiological parameter if, as an input, a motion signal is provided,
- measuring a training physiological parameter of a subject by a training physiological parameter measurement device and providing a motion signal that is related to a mechanical movement of an organ within a subject by using an RF antenna module comprising one or more RF antennas and an RF instrument connected to the RF antenna module and configured to transmit RF power into the RF antenna module, to receive an RF signal from the RF antenna module and to provide the motion signal based on the received RF signal, wherein the RF antenna module is arranged on the subject,
- determining a physiological parameter of the subject based on the model to be trained and the motion signal provided by the RF instrument and the RF antenna module and modifying the model such that a deviation between the determined physiological parameter and the training physiological parameter is reduced by a training module.

In another aspect of the present invention a computer program for controlling a measurement device configured to be worn by a subject as defined by claim 9 is presented, wherein the computer program comprises program code means for causing the measurement device to provide a motion signal that is related to a mechanical movement of a structure within the subject by using an RF instrument and an RF antenna module of the measurement device. The computer program can be configured to run on the RF instrument or on a controller of the measurement device, which is configured to control the different components of the measurement device.

In a further aspect of the present invention a computer program for controlling a determination device for determining a physiological parameter as defined by claim 10 is presented, wherein the computer program comprises program code means for causing the determination device to determine the physiological parameter based on a provided model, which provides, as an output, a physiological parameter if, as an input, a motion signal is provided, and a motion signal which has been measured by a measurement device as defined by claim 9. The computer program can be configured to run on the processor of the determination device or on a controller of the determination device, which is configured to control the different components of the determination device.

In another aspect of the present invention a computer program for controlling a training system as defined by claim 11 is presented, wherein the computer program comprises program code means for causing the training system to carry out the steps of the training method, when the computer program is run on a computer controlling the training system. The computer program can be configured to run on one or several components of the training system defined in claim 11 or on a controller of the training system, which is configured to control the different components of the training system.

It shall be understood that the system for determining a physiological parameter of a subject of claim 1, the measurement device of claim 9, the determination device of claim 10, the training system of claim 11, the method for determining a physiological parameter of a subject of claim 13, the training method, the computer program for controlling a measurement device configured to be worn by a subject of claim 14, the computer program for controlling a determination device for determining a physiological parameter of claim 15, and the computer program for controlling a training system, have similar and/or identical preferred embodiments, particularly as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: shows schematically and exemplarily an embodiment of a system for determining a physiological parameter of a subject,
- Fig. 2: shows schematically and exemplarily a measurement device of the system shown in Fig. 1, wherein the measurement device is configured to be worn by the subject,
- Fig. 3: shows schematically and exemplarily an embodiment of a determination device of the system shown in Fig. 1, wherein the determination device is configured to determine the physiological parameter based on a motion signal provided by the measurement device shown in Fig. 2,
- Fig. 4: shows schematically and exemplarily a training system fortraining a model to be used by the system shown in Fig. 1,
- Fig. 5: shows schematically and exemplarily a complex signal provided by the measurement device,
- Fig. 6: shows schematically and exemplarily the provided signal after a phase rotation has been carried out,
- Fig. 7: shows schematically and exemplarily the provided signal after a band-pass filter has been applied,
- Fig. 8: shows a flowchart exemplarily illustrating an embodiment of a method for determining a physiological parameter of a subject,
- Fig. 9: shows a flowchart exemplarily illustrating an embodiment of a training method fortraining a model to be used by the system for determining the physiological parameter of the subject, and
- Fig. 10: shows schematically and exemplarily a certain arrangement of RF antennas on a subject.

### DETAILED DESCRIPTION OF EMBODIMENTS

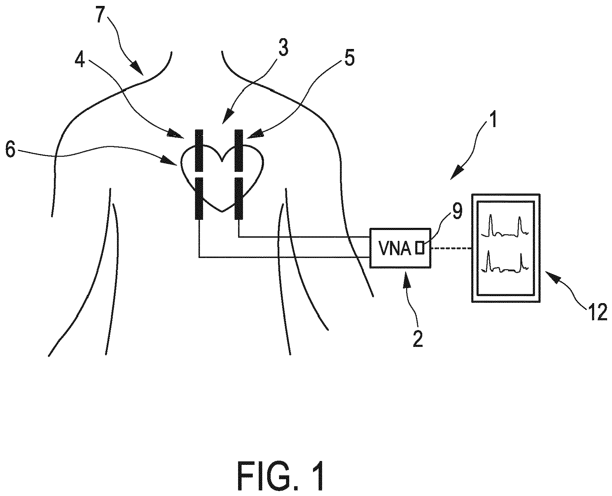

Fig. 1 illustrates schematically and exemplarily an embodiment of a system for determining a physiological parameter of a subject. In this embodiment the system 1 is configured to determine the stroke volume of the heart 6 within the subject 7 as the physiological parameter. However, the system also can be configured to determine another heart-related physiological parameter like the heart rate, or a lung-related physiological parameter like the respiratory rate.

The system 1 comprises a measurement device of which in Fig. 1 only two RF antennas 4, 5 of an RF antenna module 3 and a vector network analyzer 2 are shown. The RF antennas 4, 5 are connected to the vector network analyzer 2, wherein the vector network analyzer 2 and the RF antenna module 3, i.e. the RF antennas 4, 5, are configured to provide motion signals that are related to a mechanical movement of the heart 6 within the subject 7.

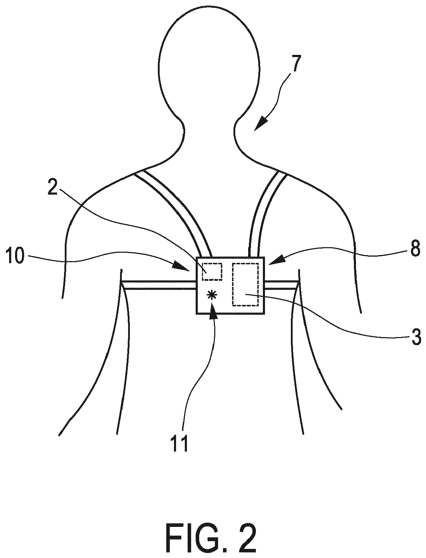

It should be noted that Fig. 1 just shows an illustration of the system 1 for determining a physiological parameter of the subject 7, wherein, for instance, the vector network analyzer 2 also is arranged on the body of the subject 7 and not somewhere in the air as shown in Fig. 1. In particular, as illustrated in Fig. 2, the measurement device 8 includes a wearable holder 10 in which the RF antenna module 3 and the vector network analyzer 2 are integrated as indicated schematically and exemplarily in Fig. 2 by dashed boxes.

The measurement device 8 further comprises a transmitter for transmitting the provided motion signals to a determination device 12 shown in Fig. 1. In this embodiment the transmitter 9 is integrated in the vector network analyzer 2 as schematically and exemplarily illustrated in Fig. 1. The transmission between the measurement device 8 and the determination device 12, particularly between the vector network analyzer 2 of the measurement device 8 and the determination device 12, is preferentially a wireless transmission like a Bluetooth transmission or any other wireless transmission.

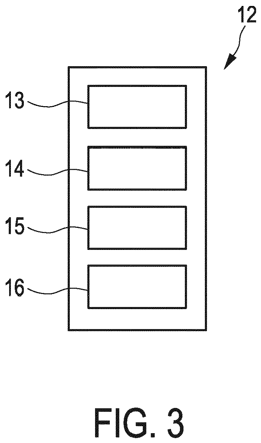

In this embodiment the determination device 12 is a smartphone or a tablet. The determination device 12 is configured to determine the physiological parameter, i.e. in this embodiment the stroke volume, based on the provided motion signals. The determination device 12 is exemplarily and schematically illustrated in Fig. 3.

The determination device 12 comprises a receiver 13 for receiving the motion signals provided by the measurement device 8. Moreover, the determination device 12 comprises a model providing module 14 configured to provide a model that provides, as an output, a physiological parameter, i.e. in this embodiment a stroke volume, if, as an input, the motion signals are provided. In this embodiment the model providing module 14 is a storage in which a correspondingly trained model is stored.

The determination device 12 further comprises a processor 15 configured to determine the physiological parameter based on the provided model and the received motion signals. The determination device 12 also comprises an output unit 16 like a display or a connector for connecting a display, in order to output the determined physiological parameter.

The wearable holder 10 comprises a visible marker 11 assigned to an anatomical feature of the subject 7, wherein the wearable holder 10 is configured to be worn such that the marker is arranged at a position on the subject 7 at which the assigned anatomical feature is located. In this embodiment the anatomical feature is the sternum at the height of the nipples, wherein, if the wearable holder 10 is worn correctly, the marker 11 coincides with the sternum at the height of the nipples.

The RF antenna module 3 has a width and a length, wherein the width and the length are each smaller than 20 cm. Moreover, in this embodiment the two RF antennas 4, 5 are dipole antennas. However, also other types of RF antennas could be used like loop coils.

The vector network analyzer 2 and the RF antenna module 3 are configured to be operated in a frequency range from 30 to 1000 MHz. In particular, the vector network analyzer 2 and the RF antenna module 3 are configured to be operated in a frequency range from 300 to 800 MHz. In a preferred embodiment the operating frequency of the vector network analyzer 2 and the RF antenna module 3 is 64, 128 or 300 MHz.

The vector network analyzer 2 is configured to provide as a motion signal a complex signal like a complex reflection coefficient signal or complex coupling coefficient signal. In this embodiment, since the RF antenna module 3 comprises two RF antennas 4, 5, the vector network analyzer 2 provides two complex signals to the determination device 12.

The processor 15 of the determination device 12 is configured to identify, for each complex signal, a first subsignal of the respective complex signal having a distinct phase shift (for example 90 degrees) with respect to a second subsignal of the respective complex signal and to determine the physiological parameter based on the separated subsignals. In this embodiment the physiological parameter is the stroke volume, wherein for this reason it desired to have a subsignal which is related to the mechanical movement of the heart 6 within the subject 7, wherein an influence by other movements within the subject 7 should be as small as possible. It has been found that in the respective complex signal the contribution caused by cardiac motion has a distinct phase shift with respect to a contribution caused by respiratory motion. Thus, by identifying the phase shift of the first subsignal with respect to a second subsignal of the respective complex signal, the influence of respiratory motion on the signal, which is finally used for determining the stroke volume, can be strongly reduced or even eliminated.

The processor 15 can be further configured to apply a blind source separation technique to separate the subsignals out of the multiple complex signals received by the antenna, for example by applying an independent component analysis or a second order blind analysis. Furthermore, the processor 15 can be configured to apply a frequency filtering to the first subsignal like a band-pass filtering, a low-pass filtering, a high-pass filtering or Kalman filtering, in order to further reduce contributions to the first subsignal, which are not caused by the mechanical movement of the heart 6.

The resulting two first subsignals, which are obtained based on the two complex signals measured by using the two RF antennas 4, 5, can be combined to a combined signal by, for example, blind source separation or principal component analysis. In another embodiment, the first subsignal with the least contribution from the second subsignal is selected based on spectral analysis.

In this embodiment the model providing module 14 is configured to provide a linear model as the model. Thus, a linear function is provided, which relates the processed signal to the stroke volume. Correspondingly, the processor 15 is configured to determine the stroke volume based on the provided linear model and the provided processed signal, wherein the provided model has been determined before by training which also could be named calibration. An embodiment of a training system for training the model to be provided by the model providing module 14 will be described exemplarily in the following.

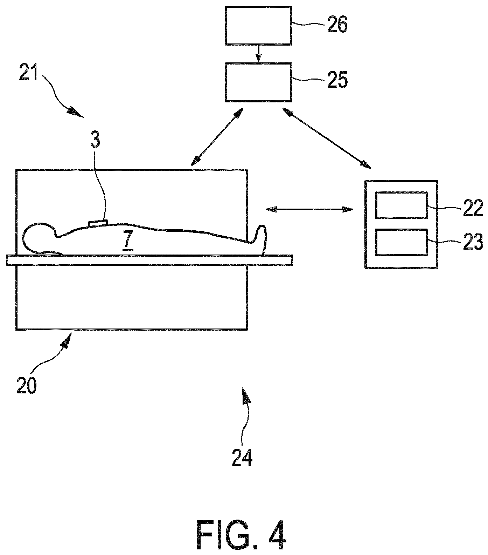

Fig. 4 shows schematically and exemplarily a training system 21 for training the model to be provided by the model providing module 14. The training system 21 comprises a training physiological parameter measurement device 24 for measuring a training physiological parameter of the subject 7 being, in this embodiment, the stroke volume.

The training physiological parameter measurement device 24 comprises an MR signals generation device 20 which uses the RF antenna module 3 of the measurement device 8 for determining the stroke volume. The training physiological parameter measurement device 24 further comprises a controller 22 for controlling the MR signals generation device 20 and a training physiological parameter determination module 23 for determining the training physiological parameter based on the generated MR signals. In this embodiment the training physiological parameter determination module 23 is configured to determine the stroke volume based on the MR signals generated by the MR signals generation device 20. For determining the stroke volume, the MR signals generation device 20, the controller 22 and the training physiological parameter determination module 23 can be configured to reconstruct MR images based on the MR signals and to determine the stroke volume based on the reconstructed MR images. In an embodiment, for determining the stroke volume, the MR signals generation device 20, the controller 22 and the training physiological parameter determination module 23 are configured to operate in accordance with known techniques like the technique disclosed in the article by Groepenhof et al., Physiological Measurements, 2007, 28(1):1-11 or in the article by Dornier et al., European Radiology, 2004 14(8):1348-52, which are herewith incorporated by reference. The MR signals generation device 20 can be a device of a standard MR system.

The training system 21 further comprises a model providing module 26 configured to provide an adaptable model to be trained, wherein the model provides, as an output, a physiological parameter, if, as an input, a motion signal is provided. In this embodiment the model is a linear model of the type SV=ax+b, where x is, for example, the amplitude of the processed signal, the amplitude of the derivative of the processed signal or another quantity derived from the processed signal. SV is the stroke volume, which preferentially is defined as the volume of blood pumped per beat from the left ventricle, and a, b are adaptable parameters which are adapted during the training process. For instance, the processed signal is the one where the first subsignal is present most strongly in the frequency domain, i.e. has the largest amplitude.

Furthermore, the training system 21 comprises the measurement device 8 configured to be worn by the subject 7, wherein for clarity reasons in Fig. 4 of the measurement device 8 only the RF antenna module 3 is shown. As mentioned above, the training physiological parameter measurement device 24 and the measurement device 8 use the same RF antenna module 3.

The training system 21 further comprises a training module 25 configured to i) determine a physiological parameter of the subject 7 based on a) the model to be trained and b) a motion signal provided by the measurement device 8 by using the RF antenna module 3 and ii) modify the model such that a deviation between this determined physiological parameter and the training physiological parameter, which has been determined by the training physiological parameter measurement device 24 by using the same RF antenna module 3, is reduced, in particular, minimized. Preferentially, the training module 25 is configured to use the same processing of the signals, which is also applied by the processor 15 before the processor 15 uses the model as described above for determining the physiological parameter during an actual determination, i.e. after the training phase has been completed. In this embodiment the training model is configured to determine the stroke volume of the subject 7 based on the model to be trained, which is preferentially a linear model, and a processed signal which has been determined as described above and to modify the model such that a deviation between this determined stroke volume and a stroke volume determined by using the MR signals generation device 20, the controller 22 and the determination device 23 is reduced, particularly minimized.

The trained model is then used by the above described system 1 for determining a physiological parameter like the stroke volume of a subject. The system can perform dynamic determinations or measurements of the physiological parameter, in particular, of the cardiac output, with the one or more RF antennas integrated into the measurement device to be worn, particularly into the wearable holder. The system 1 can be used to monitor the pumping function of the heart at home in patients with heart failure.

Generally, RF antennas transmit RF electromagnetic radiation into the surrounding and receive electromagnetic radiation from the surrounding. An RF antenna has a measurable complex impedance which quantifies the relation between the complex current and voltage at a feed port of the respective RF antenna. This antenna impedance can be derived from RF reflection measurements with the vector network analyzer. The antenna impedance changes in phase and magnitude when the surrounding of the respective RF antenna changes. This happens when an RF antenna is positioned on the body and there is motion of the heart or the lungs. This effect can be utilized to measure internal physiological motion with RF antennas. It is noted that the RF operating frequency of the system determines the range in which physiological motion is detected. For example, when operating in a frequency range up to 300 MHz, RF energy is absorbed in the whole body and global motion is measured. When operating in the range of 300 to 800 MHz, motion of organs is measured, while higher frequencies can be used to measure local motion, just centimeters under the skin, for example, if the measurement device 8 is used to measure stroke volume of the heart, it does not use RF in the GHz range, but in a range of, for instance, 300 to 800 MHz.

RF antennas are used in MRI to excite nuclear spins and detect signals emitted back by the magnetized spins. Since the MRI antennas are also sensitive to physiological motion, they can be used to detect and correct for physiological motion in MRI. This is described, for instance, in the article "The rf coil as a sensitive motion detector for magnetic resonance imaging" by D. Buikman et al., Magnetic Resonance Imaging, volume 3, pages 281 to 289 (1988) which is herewith incorporated by reference. By operating at the same frequency as MRI antennas, during the above described training the same RF antennas can be used for determining, for instance, the stroke volume by using MRI and measuring a signal by using the measurement determination device 8, thereby allowing to use MRI as a unique calibration tool. Quantitative parameters like the stroke volume of the heart can be measured simultaneously with MRI and the measurement device 8. After this training or calibration, the physiological parameter of interest like the stroke volume can be measured by using the measurement device 8 only, wherein this measurement can even be carried out at home.

The operating frequency of the respective RF antenna determines the size of the area in which the RF energy is absorbed. Based on the wavelength of the RF radiation in tissue, power is deposited in the whole body or in parts of the body. For operating frequencies within the range of 30 to 300 MHz, the RF radiation causes power deposition throughout the whole body. For higher frequencies like 300 to 1000 MHz, power is deposited only in parts of the body, for example the head. For monitoring the full cardiorespiratory system, it can be beneficial to operate in the whole body resonant area like 30 to 300 MHz, or a frequency range in which resonance occurs only in the thorax. For monitoring smaller organs such as the heart, higher operating frequencies are desirable like 300 to 800 MHz. For monitoring local motion of small structures, even higher operating frequencies like 800 to 1200 MHz are of interest. Generally, higher frequencies can be used to identify local motion of small structures, while lower frequencies can be used to identify motion of larger structures like full organs The used operating frequency therefore depends on the motion of which structure should be used for determining a respective physiological parameter. In the above described embodiment in which the stroke volume should be determined, the operating frequency is preferentially equal to the Larmor frequency of widely available MRI systems, i.e. 64 MHz for 1.5 T systems, 128 MHz for 3 T systems and 300 MHz for 7 T systems. This allows to use the same RF antenna for MRI and for the measurement carried out by the measurement device 8.

In an embodiment, RF frequency sweeps are performed, in which the operating frequency of the measurement device is changed over time. For example, by rapidly switching between measurements at 64 and 600 MHz, information about whole body movement and organ movement can be obtained in a single measurement. When this is done, it should be ensured that the sample rate at the respective frequencies remains higher than two times the respective frequency to satisfy the Nyquist criterion.

In an embodiment the RF antennas can be loop antennas, which are commonly used in MRI, where they function as transmit and/or receive antennas. This enables simultaneous MRI and measurements with the measurement device.

Especially for measurements on the cardiorespiratory system, the processor is configured to achieve a separation of cardiac and respiratory signals, i.e. into a first subsignal being heart-related and a second subsignal being lung-related. Particularly if the measurement device is used for carrying out a complex reflection measurement, the resulting cardiac and respiratory signals are periodic and have a distinct phase difference, for example 90 degrees. In a preferred embodiment, the processor is configured to perform a phase rotation such that the cardiac signal, i.e. the first subsignal, appears on the real axis and the respiratory signal, i.e. the second subsignal, mostly on the imaginary axis. To be more generic, the processor can be configured to perform a transformation, in particular a 2x2 matrix transformation, on the complex signals measured by the measurement device 8 to achieve this.

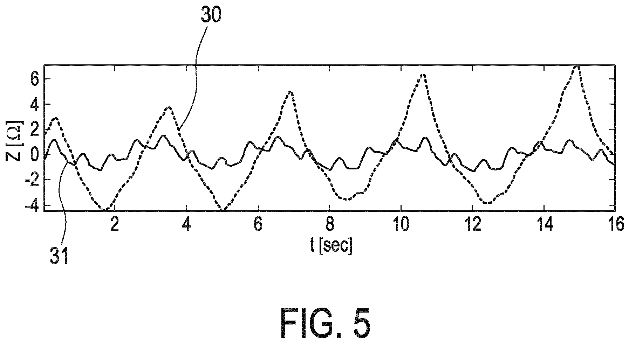

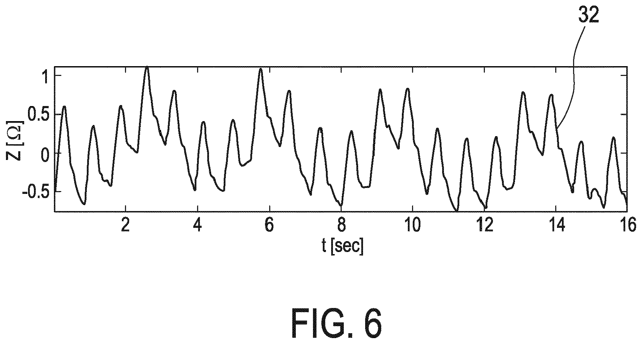

Fig. 5 schematically and exemplarily illustrates a measured complex signal Z over time, wherein in this figure the curve 30 is the imaginary part of the signal Z and the curve 31 is the real part of the signal Z. Fig. 6 illustrates the signal Z after the phase rotation has been carried out as described above, i.e. it shows the first subsignal along the real axis.

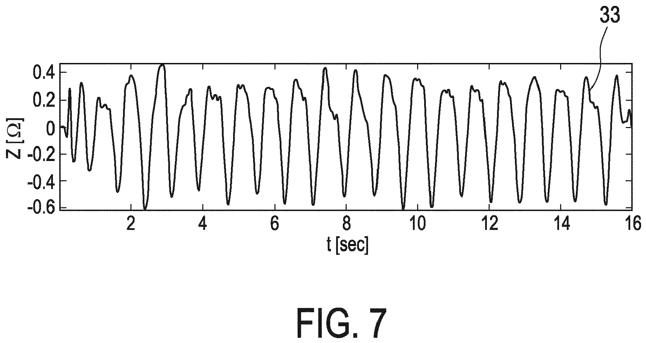

The processor can be further configured to exploit differences in spectral characteristics, in order to remove remaining contributions of motion that are not of interest. Thus, the processor can be configured to perform filtering in the frequency domain. For instance, a band-pass filter, a low-pass filter, a high-pass filter or a Kalman filtering could be used. In an embodiment, a band-pass filter between 0.75 and 10 Hz is used, in order to filter out remaining components of a respiratory signal. This is illustrated in Fig. 7. Thus, in Fig. 7 the curve 33 results from filtering the curve 32 shown in Fig. 6 by using a band-pass filter between 0.75 and 10 Hz.

A model can be used to predict stroke volume (SV, mL) from the measurement shown in Fig. 7. For example, if the amplitude of the signal, the amplitude of the derivative of the signal or any other quantity derived from the signal in Fig. 7 is given as x, the stroke volume can be calibrated through a linear relationship as SV=a*x+b, where a and b are determined in a calibration phase. In the calibration phase, the stroke volume is measured with a reference instrument such as MRI or ultrasound. This can be done under physiological stress, in order to increase the stroke volume during the measurements. At the same time, the parameter x is derived from the signal measured by using the RF antenna and the vector network analyzer. If SV and x are available for several different values of SV, the parameters a and b can be determined.

The above described system 1 can be used, for instance, to monitor the heart's pumping function in patients with heart failure at home. It can also be used to measure the heart rhythm and arrhythmia, or to quantify lung ventilation or edema, particularly to locally quantify lung ventilation or edema at home. For measuring the heart's pumping function, the stroke volume could be predicted based on a model of the effect of stroke volume on the measurements, for example the linear model SV=a*x+b. The same is possible for tidal volume, where the tidal volume (TV in mL) can be determined as TV = c*y+d, where y can be the amplitude of the respiration signal, and c and d are model parameters derived during a calibration measurement with a reference instrument such as spirometry. The TV and y can be measured during physiological stress, this will result in increasing TV over time. Based on this measurement, the parameters c and d can be determined. Parameters such as heart rate or respiratory rate can be derived from frequency domain analysis of the combined signals.

It is also possible to track catheters during heart catheterizations and to monitor, for instance, heart-related and/or lung-related physiological parameters in sports.

RF antennas can emit and detect electromagnetic radiation, wherein an RF impedance that is measured by an antenna changes based on the surrounding of the antenna. If an RF antenna is placed on a body and the dielectric properties of the body change, the RF impedance will also change. The dielectric properties of the body change during mechanical motion of, for example, the heart or the lungs, and they also change when external structures such as catheters are moving through the body. This enables the determination of, for instance, heart-related and/or lung-related physiological parameters based on motion of a structure of the subject like an organ or on motion of another structure like a catheter.

The RF antennas of the RF antenna module preferentially are flexible and lightweight, i.e. preferentially they have a weight being smaller than 30 g. In an embodiment, the RF antennas are integrated or even sewn in clothing. To measure RF back scattering with these RF antennas, the vector network analyzer is connected to the RF antennas. The vector network analyzer preferentially is a small mobile device which could even be held in a hand. The vector network analyzer is preferentially also integrated in the clothing. In particular, the above described wearable holder 10 does not only comprise the RF antennas of the RF antenna module, but also the vector network analyzer 2. However, in an embodiment it is also possible that the holder 10 only includes the RF antennas and that the vector network analyzer is held on the body by another means like a second holder.

As described above, the model can be trained such that it relates RF measurements carried out by the measurement device 8 to parameters obtained from MRI, wherein in the above described embodiment the physiological parameter is the stroke volume. To generate this model, a calibration step is performed, in which simultaneous MR imaging and RF measurements are done. Since the same one or several RF antennas are used for the MRI measurement and the RF measurement, the calibration can be integrated into an MRI process rather simply.

Although above a correlation with an output of an MRI measurement is described, the training or calibration could also be carried out with other kinds of measurements, i.e. with other calibration measurements, in order to correlate these other measurements like computed tomography (CT) or ultrasound measurements, i.e. physiological parameters obtained from these other measurements, to the RF measurements carried out by the measurement device which finally uses the correspondingly trained or calibrated model.

Generally, the model allows to provide a relation between a) the RF measurement carried out by using the measurement device, particularly the motion signal generated by using the RF measurement, and b) one or several physiological parameters obtained from an even relatively complex imaging modality like MRI or CT, wherein this relation can be used together with the RF measurements carried out by the measurement device, in order to determine a physiological parameter which normally would require a relatively complex imaging modality.

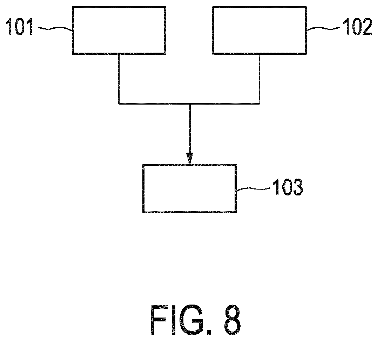

In the following an embodiment of a method for determining a physiological parameter of a subject will be described with reference to a flowchart shown in Fig. 8.

In step 101, a motion signal is provided, which is related to a mechanical movement of an organ like the heart within the subject by using a vector network analyzer and an RF antenna module of the measurement device 8. In step 102, a model is provided, wherein the model has been trained to provide, as an output, a physiological parameter if, as an input, a motion signal is provided. The model is provided by the model providing module 14. In step 103, the physiological parameter is determined based on the provided model and the provided motion signal by the processor 15.

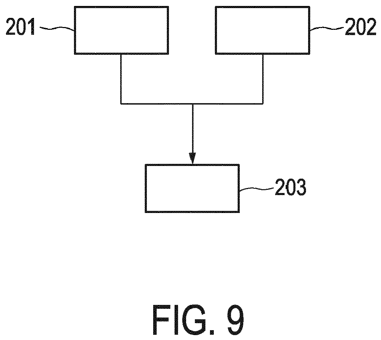

In the following an embodiment of a training method for training a model to be provided by the model providing module 14 will be exemplarily described with reference to a flowchart shown in Fig. 9.

In step 201, a training physiological parameter of a subject is measured by the training physiological parameter measurement device 24. For instance, by using MRI, a stroke volume of a heart is determined as the training physiological parameter. At the same time, a motion signal is provided, which is related to a mechanical movement of an organ within a subject, by using the measurement device 8. In particular, complex RF signals are measured, which are related to the mechanical movement of the heart. In step 202, a model to be trained is provided by a model providing module, wherein the model provides, as an output, a physiological parameter if, as an input, a motion signal is provided. In step 203, a physiological parameter of the subject is determined based on the model to be trained and the motion signal provided by the measurement device and the model is modified such that a deviation between the determined physiological parameter and the training physiological parameter is reduced, wherein this step is carried out by the training module 25. For instance, the model can be adapted such that a deviation between a stroke volume measured by the training physiological parameter measurement device 24 and a stroke volume determined by using the signal measured by the measurement device 8 and the model to be trained is reduced, particularly minimized.

Although in above described embodiments the RF antenna module has two RF antennas, the RF antenna module can also have a single RF antenna only or more than two RF antennas.

For instance, in an embodiment the RF antenna module comprises one RF antenna and the measurement device is configured such that, if the measurement device is worn by an adult, a center point of the RF antenna is positioned left to the sternum. This single RF antenna can be positioned with a shift within the range of 2 cm to 4 cm, further preferred with a shift of 3 cm, to the left of the sternum, in order to allow the measurement device to accurately measure a signal that is related to the movement of the heart.

If in another embodiment the RF antenna module comprises two RF antennas, these two RF antennas can be arranged such that they are positioned close to the heart, but at the same time positioned relatively far away from each other, in order to have a relatively low inter-element coupling. Such a configuration is schematically and exemplarily illustrated in Fig. 1. Thus, the two RF antennas can be arranged above the edges of the heart or above edge regions of the heart, wherein these edge regions could be left/right edge regions or top/down edge regions. Correspondingly, the distance between the two RF antennas is similar to a distance between these edge regions, wherein the dimensions of the heart and hence the distance between the edge regions can be determined beforehand by using an imaging modality like MRI or CT, or standard dimensions of a human heart of an adult or child, depending on the age of the subject, can be used for defining the arrangement of the two RF antennas.

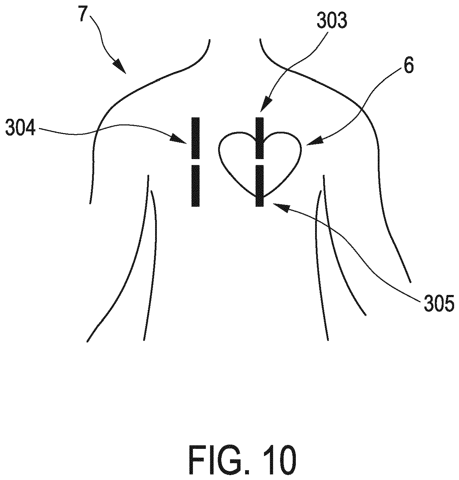

In a further embodiment, the RF antenna module 303 also comprises two RF antennas 304, 305, wherein the measurement device is configured such that, if the measurement device is worn by an adult, a center point of one 305 of the two RF antennas is positioned left to the sternum and a center point of the other 304 of the two RF antennas is positioned right to the sternum. This is schematically and exemplarily illustrated in Fig. 10. In particular, one of the RF antennas is positioned with a shift within the range of 2 cm to 4 cm, further preferred with a shift of 3 cm, to the left of the sternum and the other of the RF antennas is positioned with a shift within the range of 2 cm to 4 cm, further preferred with a shift of 3 cm, to the right of the sternum.

The holder can be anything which holds the RF antenna module on the body of the subject. It can be any wearable like a shirt, a band, et cetera, wherein especially for this reason the one or more RF antennas are preferentially flexible.

The vector network analyzer is portable and used to measure back scattering, i.e. the motion signal that is related to the mechanical movement of the organ like the heart. The measured signal preferentially is complex, i.e. it has a phase and a magnitude, wherein the vector network analyzer sends data representing the measured signal wirelessly to the determination device such as a personal computer or a mobile device, wherein the wireless data connection could be, for instance, Bluetooth or another wireless data connection.

The system for determining the physiological parameter of the subject can be configured to remotely monitor the heart function. For instance, heart failure can be monitored directly. Heart failure is a defect in the pumping function of the heart, for instance, the heart is not able to pump sufficient blood into the surrounding tissue which can lead to symptoms such as lung edema, sudden weight increase, tiredness and ultimate damage to the heart and other tissues. After a first treatment in a hospital, heart failure patients are very often re-hospitalized when symptoms of heart failure reoccur. Over 50 % of all heart failure patients are re-hospitalized after six months of initial treatment. Heart failure is the leading cause of hospitalization in adults over 65 years in the U.S. Reoccurrence of heart failure is noticed when patients show symptoms, which is already too late, by then the function of the heart has deteriorated further. By using the above described system for determining a physiological parameter of a subject, it is possible to measure heart failure before symptoms occur, wherein the patient's medication or lifestyle then can be adapted to prevent re-hospitalization. With known systems it is not possible to measure the heart pumping function remotely and with enough accuracy. For example, a common remote measurement method for heart rhythm, electrocardiography (ECG), measures only neurological impulses, but not the actual mechanical response of the heart to these impulses. ECG only measures heart rhythm, but not the heart pumping function. Since the above described system for determining a physiological parameter of a subject is sensitive to tissue deformation and changes in blood volume, the system can be used to sense changes in the heart pumping function, unlike ECG which is not directly sensitive to this.

The system also can be configured to monitor cardiac failure indirectly through detection of lung edema. Cardiac failure patients often suffer from lung edema as a result of cardiac failure. If the patients show symptoms of lung edema, there is already significant damage done to the heart and lungs. The system can be configured such that the provided motion signal is related to the mechanical movement of the lungs within the subject, wherein in this case the signal is very sensitive to respiratory motion. Since with developing lung edema the motion of the lungs changes, by monitoring the movement of the lungs, developing lung edema can be detected, thereby indirectly detecting cardiac failure. In this example, the determined physiological parameter can be a characteristic of the movement of the lungs like the frequency or amplitude of this movement.

If the system is configured to provide a motion signal that is related to a mechanical movement of the heart within a subject and to use this signal to determine a heart-related physiological parameter like the stroke volume or the heart rate, the heart-related physiological parameter can be used to monitor arrhythmia in cardiovascular patients. Such monitoring is normally done by using ECG measurements. However, ECG measurements use electrodes that are attached to the skin which is uncomfortable for patients. The above described system for determining a heart-related physiological parameter of the subject does not need to be attached to the skin, thus improving patient comfort.

The system can also be configured to remotely monitor lung ventilation. In particular, the measurement device can be configured to provide a motion signal that is related to the mechanical movement of the lungs within a subject, wherein the model can be trained such that, given the motion signal, a lung-related physiological parameter measured by, for instance, spirometry or MRI is output. The processor of the determination device then can determine a lung-related physiological parameter based on the provided motion signal and the trained model. In this case, for training the model a spirometry system or MRI system can be used.

The system can also be used for tracking catheters during implantation. During heart catheterization, generally a long thin tube is inserted in an artery or vein and threaded to the heart where it is used to treat or diagnose certain heart diseases. These catheters contain electrically conductive materials which makes RF measurements very sensitive to the position and movement of these wires. The resulting motion signal, which is related to the mechanical movement of the catheters, can be used for determining a physiological parameter like the stroke volume.

The system can also be configured to measure a heart-related physiological parameter like the heart rate or a lung-related physiological parameter like the breathing rate during physical exercise. It is known to do this with ECG which needs to make contact with the skin by using electrodes. In contrast to this, the above described system can measure the heart-related or lung-related physiological parameters without needing to make contact with the skin.

In a preferred embodiment in which the RF antenna module comprises two RF antennas only, the vector network analyzer and the two RF antennas can be configured such that an inter-element coupling between the two RF antennas is below a predefined value, wherein this predefined value might be, for instance, -12 dB. In particular, the distance between the two RF antennas is such that the inter-element coupling between the two RF antennas is below -12 dB. This distance can be, for instance, within a range of 4 to 8 cm and preferentially is 6 cm, wherein the distance refers to the distance between the center positions of the two RF antennas. The inter-element coupling might be measured with the two antennas and the RF instrument, which preferentially is a vector network analyzer, by quantifying the magnitude and phase of the signal that is reflected into antenna 2 when antenna 1 is transmitting.

In an embodiment the RF antenna module includes at least a first RF antenna and a second RF antenna, wherein the vector network analyzer and the RF antenna module are configured to measure a first motion signal of the first RF antenna that is related to a mechanical movement of a first organ within the subject and to measure a second motion signal of the second RF antenna that is related to a mechanical movement of a second organ within the subject, wherein the processor is configured to determine a coupling between the first RF antenna and the second RF antenna, to remove contributions of the movement of the second organ to the first motion signal from the first motion signal based on the determined coupling and to determine the physiological parameter based on the first motion signal. For instance, an antenna 1 can be positioned close to an organ of interest such that the reflection of antenna 1 is mainly affected by motion of this organ. An antenna 2 can be placed further away from the organ of interest and closer to another organ that can cause distortion in the signal of antenna 1, for example antenna 2 can be positioned closer to the lungs if the heart is of interest. The coupling between antenna 1 and antenna 2 will be more affected by motion of the organ that causes distortions, wherein the coupled signal from antenna 1 and 2 can be used to remove distortion from the signal of interest measured in antenna 1. To remove these artifacts, techniques such as blind source separation or second order blind analysis can be used.

Moreover, in an embodiment, the RF antenna module includes several RF antennas having different transmit phases defining a sensitivity profile of the RF antenna module, wherein the RF antenna module is configured such that the sensitivity profile has its largest sensitivity at the location of the organ. For instance, the RF antenna module might comprise several RF antennas which are arranged in a belt-like configuration. The belt-like configuration can be such that the RF antennas are arranged around a torso of the subject. Preferentially, the number of RF antennas of this belt-like configuration is within a range from 3 to 32 RF antennas.

Although in above described embodiments the model mainly is a linear model, the model can also be another one. Generally, the model can be any relation between a) a physiological parameter like the stroke volume or a ventilation parameter and b) the motion signal provided by the measurement device. Such a relation could be determined by calibration/training, but also by electromagnetic simulation. For instance, for different distributions and dimensions of human components like organs, bones, skin, et cetera a respective electromagnetic simulation can be carried out and hence a respective relation, i.e. model, can be determined. Based on a specific distribution and specific dimensions of, for instance, the organs, the bones, the skin, et cetera of a respective subject, which might be known based on an image of the respective subject like an MRI, CT, ultrasound et cetera image, a matching model can be selected and used for determining the physiological parameter based on the motion signal. For carrying out the electromagnetic simulation, finite difference time domain simulations can be used. This can be done with commercially available electromagnetic solvers such as shown in the article by Navest et al., Magnetic Resonance in Medicine, 2019, 82:6 (2236-2247) which is herewith incorporated by reference.

In an embodiment, the relations and hence the models, which have been determined by electromagnetic simulation, together with body parameters describing the respective distributions and dimensions of human components like organs, bones, skin, et cetera can be used to train an artificial intelligence (Al). The body parameters could be, for instance, a dimension of the torso like its circumference and the AI can be trained such that, given one or several body parameters and the motion signal provided by the measurement device, the physiological parameter is output. Different AI methods could be used, for example regression models, Gaussian processes, neural networks, k-nearest neighbors or support vector machine. In an embodiment, scalar parameters such as body circumference, stroke volume at rest, BMI, age or sex are specified as input to the model. Moreover, in an embodiment, a model of the dielectric property distribution in the area of interest like the torso of the subject is obtained based on MRI, CT or ultrasound imaging. The dielectric property distribution can be provided as an input to train the AI and later to update the model.

In a further embodiment a specific distribution and specific dimensions of human components like organs, bones, skin, et cetera of the subject, of whom the relation between the motion signal provided by the measurement device and the physiological parameter should be determined, are determined based on an image of the subject like a CT or MR image, wherein the relation, i.e. the model, can be determined based on an electromagnetic simulation applied to the determined specific distribution and specific dimensions of the human components.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the determination of the physiological parameter, the training of the model, et cetera performed by one or several units or devices can be performed by any other number of units or devices. These procedures and/or the control of the components of the system for determining the physiological parameter of the subject in accordance with the above described method for determining the physiological parameter of the subject and/or the control of the training system in accordance with the training method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A system for determining a physiological parameter of a subject (7), the system (1) comprising:
- a measurement device (8) configured to be worn by the subject (7) and including a) an RF antenna module (3) comprising one or more RF antennas (4, 5) and b) an RF instrument (2) connected to the RF antenna module (3) and configured to transmit RF power into the RF antenna module (3), to receive an RF signal from the RF antenna module (3) and to provide a motion signal that is related to a mechanical movement of a structure (6) within the subject (7) based on the received RF signal,
- a determination device (12) configured to determine the physiological parameter based on the provided motion signal, wherein the determination device (12) comprises a model providing module (14) configured to provide a model that provides, as an output, a physiological parameter if, as an input, a motion signal is provided, and a processor (15) configured to determine the physiological parameter based on the provided model and the provided motion signal.

2. The system as defined by any of the preceding claims, wherein the RF instrument (2) is configured to provide as the motion signal a complex signal.

3. The system as defined by claim 2, wherein the processor (15) is configured to identify a first subsignal of the complex signal having a distinct phase shift with respect to a second subsignal of the complex signal and to determine the physiological parameter based on at least one of the subsignals.

4. The system as defined by any of the preceding claims, wherein the RF instrument (2) and the RF antenna module (3) are configured to be operated in a frequency range from 30 to 1000 MHz.

5. The system as defined by claim 4, wherein the RF instrument (2) and the RF antenna module (3) are configured to be operated with an operating frequency of 64, 128 or 300 MHz.

6. The system as defined by any of the preceding claims, wherein the RF antenna module (3) includes at least a first RF antenna and a second RF antenna, wherein the RF instrument (2) and the RF antenna module (3) are configured to provide a first motion signal of the first RF antenna that is related to a mechanical movement of a first structure within the subject (7) and to measure a second motion signal of the second RF antenna that is related to a mechanical movement of a second structure (6) within the subject (7), wherein the processor (15) is configured to remove contributions of the movement of the second structure (6) to the first motion signal from the first signal based on the provided first and second motion signals and to determine the physiological parameter based on the first signal.

7. The system as defined by any of the preceding claims, wherein the RF antenna module (3) includes several RF antennas (4, 5) having different transmit phases defining a sensitivity profile of the RF antenna module (3), wherein the RF antenna module (3) is configured such that the sensitivity profile has its largest sensitivity at the location of the structure (6).

8. The system as defined by any of the preceding claims, wherein the measurement device (8) includes a wearable holder (10) comprising at least the one or more RF antennas (4, 5) of the RF antenna module (3), wherein the wearable holder (10) comprises a visible marker (11) assigned to an anatomical feature of the subject (7), wherein the wearable holder (10) is configured to be worn such that the marker is arranged at a position on the subject (7) at which the assigned anatomical feature is located.

9. A measurement device configured to be worn by a subject (7), wherein the measurement device (8) is configured to be used with the determination device (12) defined by claim 10 for forming the system for determining a physiological parameter as defined by claim 1, wherein the measurement device (8) includes a) an RF antenna module (3) comprising one or more RF antennas (4, 5) and b) an RF instrument (2) connected to the RF antenna module (3) and configured to transmit RF power into the RF antenna module (3), to receive an RF signal from the RF antenna module (3) and to provide a motion signal that is related to a mechanical movement of a structure (6) within the subject (7).

10. A determination device for determining a physiological parameter of a subject based on a motion signal measured by the measurement device defined by claim 9, wherein the determination device (12) comprises a model providing module (14) configured to provide a model that provides, as an output, a physiological parameter if, as an input, a motion signal is provided, and a processor (15) configured to determine the physiological parameter based on the provided model and the provided motion signal.

11. A training system for training a model to be used by a system for determining a physiological parameter of a subject (7) as defined by any of claims 1 to 8, wherein the training system (21) comprises:
- a training physiological parameter measurement device (24) for measuring a training physiological parameter of a subject (7),
- a model providing module (26) configured to provide an adaptable model to be trained, wherein the model provides, as an output, a physiological parameter if, as an input, a motion signal is provided,
- an RF antenna module (3) comprising one or more RF antennas (4, 5) and an RF instrument (2) connected to the RF antenna module (3) and configured to transmit RF power into the RF antenna module (3), to receive an RF signal from the RF antenna module (3) and to provide a motion signal that is related to a mechanical movement of a structure (6) within the subject (7) based on the received RF signal, if the RF antenna module (3) is arranged on the subject (7),
- a training module (25) configured to a) determine a physiological parameter of the subject (7) based on the model to be trained and a motion signal provided by the RF instrument (2) and the RF antenna module (3) and b) modify the model such that a deviation between the determined physiological parameter and the training physiological parameter is reduced.

12. The training system as defined by claim 11, wherein the training physiological parameter measurement device (24) is configured to use the RF antenna module (3) for measuring the training physiological parameter of the subject.

13. A method for determining a physiological parameter of a subject (7), the method (1) comprising:
- providing a motion signal that is related to a mechanical movement of a structure (6) within the subject (7) by using an RF instrument (2) and an RF antenna module (3) of a measurement device as defined by claim 9,
- providing a model that provides, as an output, a physiological parameter if, as an input, a motion signal is provided, by a model providing module (14), and
- determining the physiological parameter based on the provided model and the provided motion signal by a processor (15).

14. A computer program for controlling a measurement device configured to be worn by a subject (7) as defined by claim 9, wherein the computer program comprises program code means for causing the measurement device to provide a motion signal that is related to a mechanical movement of a structure (6) within the subject (7) by using an RF instrument (2) and an RF antenna module (3) of the measurement device.

15. A computer program for controlling a determination device (12) for determining a physiological parameter as defined by claim 10, wherein the computer program comprises program code means for causing the determination device (15) to determine the physiological parameter based on a provided model, which provides, as an output, a physiological parameter if, as an input, a motion signal is provided, and a motion signal which has been provided by a measurement device as defined by claim 9.
